(19) Europäisches Patentamt
European Patent Office
Office européen des brevets

(11) Veröffentlichungsnummer: **0 464 642 A1**

# (12) EUROPÄISCHE PATENTANMELDUNG

(21) Anmeldenummer: **91110574.0**

(51) Int. Cl.[5]: **C07H 19/06**, A61K 31/70

(22) Anmeldetag: **26.06.91**

(30) Priorität: **02.07.90 DE 4021006**

(43) Veröffentlichungstag der Anmeldung:
**08.01.92 Patentblatt 92/02**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Uhlmann, Eugen, Dr.**
**Zu Talblick 31**
**W-6246 Glashütten(DE)**
Erfinder: **Peyman, Anuschirwan, Dr.**
**14 Sumner Road**
**Brookline, MA 02146(US)**
Erfinder: **Winkler, Irvin, Dr.**
**In den Eichen 40**
**W-6237 Liederbach(DE)**
Erfinder: **Meichsner, Christoph, Dr.**
**Bienerstrasse 30**
**W-6238 Hofheim am Taunus(DE)**
Erfinder: **Helsberg, Matthias, Dr.**
**Am Rosengarten 3**
**W-6233 Kelkheim (Taunus)(DE)**

## (54) Pyrimidin-Derivate, deren Herstellung und Verwendung sowie diese enthaltende Arzneimittel.

(57) Verbindungen der Formel I

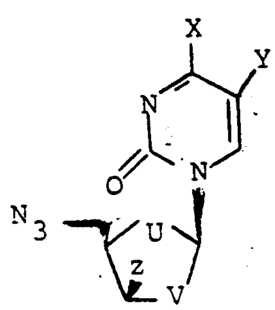

I,

in der die Symbole U-Y die genannten Bedeutungen haben, besitzen eine antivirale Wirksamkeit.

EP 0 464 642 A1

Die vorliegende Erfindung betrifft Pyrimidin-Derivate, deren Herstellung und Verwendung sowie diese Stoffe enthaltende Arzneimittel.

Es ist bereits bekannt, daß bestimmte Thymidin-Derivate, wie z.B. das AZT (3'-Azido-3'-desoxythymidin) eine antivirale Wirksamkeit aufweisen. Weiterhin ist bereits bekannt, daß weitere Thymidin-Derivate sowie Uridin- und Cytidin Derivate (vgl. DE-OS 36 43 091 A1) ebenfalls eine antivirale Aktivität aufweisen. Überraschenderweise wurde nun gefunden, daß in bestimmter Weise substituierte Azido-Xylofuranosyl- und Azido-nor-Xylofuranosyl-pyrimidin-Derivate ebenfalls eine antivirale Wirksamkeit besitzen.

Erfindungsgegenstand sind demzufolge Verbindungen der Formel I

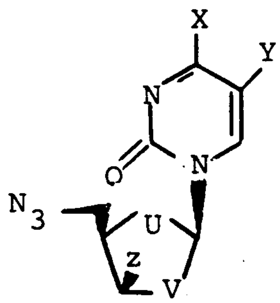

(I)

in der

X = H, OH, $OR^1$, $NH_2$, $NH-CO-R^2$, $N-CO-R^2(CO-R^3)$
Y = H, $C_1$-$C_4$-Alkyl, Halogen
Z = OH, $N_3$, F
U = O, S, $CH_2$, $CF_2$, CHF
V = O, S, $CH_2$ oder eine direkte Bindung $C_1$-$C_3$

bedeuten, wobei $R^1$ - $R^3$ unabhängig voneinander geradkettige oder verzweigte Alkylgruppen mit 1 bis 20 Kohlenstoffatomen, Cycloalkylgruppen mit 3 bis 8 Kohlenstoffatomen, Aralkylgruppen mit 7 bis 20 Kohlenstoffatomen, Arylgruppen mit 6 bis 14 Kohlenstoffatomen sind, wobei $R^2$ und $R^3$ gegebenenfalls zusammen eine cyclische Imidstruktur bilden können.

Bevorzugt sind Verbindungen der Formel I in der

X = H, OH, $OR^1$, $NH_2$, $NH-CO-R^2$, $N-CO-R^2$ $(CO-R^3)$
Y = H, $C_1$-$C_4$-Alkyl, Halogen
Z = OH, $N_3$, F
U = O, S, $CH_2$, $CF_2$, CHF
V = $CH_2$

bedeuten, wobei $R^1$ - $R^3$ geradkettige oder verzweigte Alkylgruppen mit 1-6 C-Atomen, Cycloalkylgruppen mit 3-6 C-Atomen oder Aralkylgruppen mit 7-10 C-Atomen sind, wobei $R^2$ und $R^3$ gegebenenfalls zusammen eine cyclische Imidstruktur bilden können.

Besonders bevorzugt sind Verbindungen der Formel I in der

X = H, OH, $NH_2$
Y = H, $CH_3$, $C_2H_5$, Cl
Z = OH, $N_3$
U = O, $CH_2$
V = $CH_2$

bedeuten,

insbesondere Verbindungen der Formel I in der

X = OH, $NH_2$
Y = H, $CH_3$, Cl
Z = OH, $N_3$
U = O, $CH_2$
V = $CH_2$

bedeuten.

Unter der Bezeichnung Alkylgruppe sollen beispielsweise folgende Reste verstanden werden: Methyl, Ethyl, Propyl, Isopropyl, n-Butyl, sec.-Butyl, tert.-Butyl, 2,2-Dimethyl-1-propyl, n-Pentyl, n-Hexyl, n-Heptyl, n-Octyl, n-Nonyl und n-Decyl. Unter der Bezeichnung Cycloalkylgruppe sind Beispielsweise Cyclopropyl, Cyclobutyl, Cyclopentyl oder Cyclohexyl zu verstehen. Unter der Bezeichnung Aralkylgruppe sind beispielsweise folgende Reste zu verstehen: Phenylmethyl, Phenylethyl, Phenylpropyl, Phenylbutyl, Phenylpentyl, Phenylhexyl, Phenylheptyl, Phenyloctyl, Phenylnonyl oder Phenyldecyl.

Verbindungen der Formel I von ganz besonderer Bedeutung sind:

(A) 1-(5-Azido-2,3-didesoxy-β-D-xylofuranosyl-)thymin
(B) 1-(5-Azido-2,3-didesoxy-β-D-xylofuranosyl-)cytosin
(C) 5-Chlor-1-(5-azido-2,3-didesoxy-β-D-xylofuranosyl-)cytosin
(D) 1-(3,5-Diazido-2,3,5-tridesoxy-β-D-xylofuranosyl-) thymin
(E) 1-(3,5-Diazido-2,3,5-tridesoxy-β-D-xylofuranosyl-) cytosin
(F) 5-Chlor-1-(3,5-diazido-2,3,5-tridesoxy-β-D-xylofuranosyl-)cytosin

Tautomere Formen obiger Verbindungen sind ebenfalls Gegenstand der Erfindung.

Zum Gegenstand der vorliegenden Erfindung gehört weiterhin ein Verfahren zur Herstellung von Verbindungen der Formel I, das dadurch gekennzeichnet ist, daß zur Herstellung der 5-Azido-3-hydroxy-β-D-xylofuranosyl-Derivate eine Verbindung der Formel II

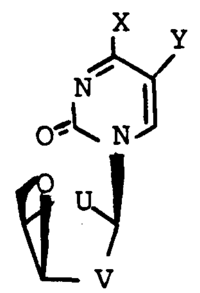

(II)

in der die Symbole die obengenannten Bedeutungen haben, mit Azid umgesetzt wird, oder zur Herstellung der 3,5-Diazido-β-D-xylofuranosyl-Derivate eine Verbindung der Formel III

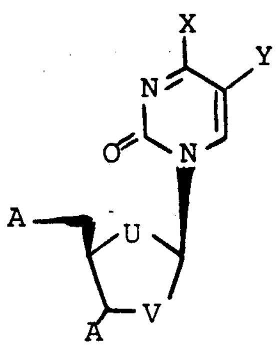

(III)

in der die Substituierten U-Y die obengenannten Bedeutungen haben und A eine Abgangsgruppe darstellt, mit Azid umgesetzt wird
oder zur Herstellung von 5-Azido-3-fluoro-β-D-xylofuranosyl-Derivateneine Verbindung der Formel IV

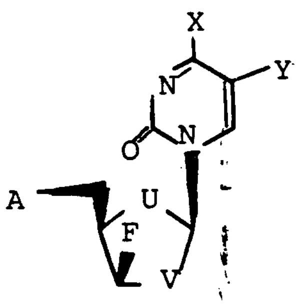

(IV)

in der die Symbole U-Y die obengenannten Bedeutungen haben und A eine Abgangsgruppe darstellt, mit Azid umgesetzt wird.

Die Abgangsgruppe A in den Verbindungen der Formel III und IV kann unterschiedlicher Natur sein; besonders bevorzugte Abgangsgruppen sind Alkylsulfonsäureester-Reste wie z.B. der Mesyl-Rest oder Arylsulfonsäureester-Reste wie z.B. der Tosyl-Rest.

Zur erfindungsgemäßen Umsetzung sind unterschiedliche Azide geeignet. Besondere Bedeutung besitzen Alkali-Azide, insbesondere Lithiumazid.

Es ist zweckmäßig, zur Durchführung der Reaktion das betreffende Azid in einem Überschuß gegenüber der Verbindung der Formel II, III oder IV umzusetzen; besonders geeignet ist ein 10-30facher Überschuß.

Die erfindungsgemäße Reaktion wird vorzugsweise in einem wasserfreien inerten Lösungsmittel durchgeführt, ein besonders bevorzugtes Lösungsmittel ist DMF.

Die bevorzugte Reaktionstemperatur liegt bei 20-150 °C, besonders bevorzugt ist ein Temperaturbereich zwischen 75 und 130 °C. Es kann nötig sein, die optimale Reaktionstemperatur und Reaktionszeit unter speziellen Reaktionsbedingungen empirisch zu ermitteln.

Die Aufarbeitung der erfindungsgemäßen Produkte kann nach üblichen Verfahren wie z.B. durch Umkristallisation oder chromatographischen Methoden erfolgen.

Die für das erfindungsgemäße Verfahren notwendigen Ausgangsprodukte lassen sich wie z.B. in Biochemical Pharmacology, Vol. 36, No. 17, S. 2713-2718, 1987, J. Org. Chem., Vol. 39, No. 11, 1974 und J. Med. Chem. 1987, 30, 2131 - 2137 beschrieben herstellen.

Die erfindungsgemäßen Verbindungen haben eine pharmazeutische Wirkung; aus diesem Grund gehört ihre Verwendung im Arzneimittel-Bereich ebenfalls zum Gegenstand der vorliegenden Erfindung. Besondere Bedeutung besitzen die erfindungsgemäßen Verbindungen bei Prophylaxe und Behandlung von durch DNA- oder RNA-Viren verursachten Krankheiten, insbesondere bei Krankheiten verursacht durch HIV, FLV, Myxo-Viren (z.B. Influenza A2 Virus), Herpes Simplex Viren und Cytomegalie-Viren.

Hauptanwendungsgebiet der erfindungsgemäßen Verbindungen sind durch Viren bedingte Erkrankungen des Respirationstraktes, der Haut, der Augen und des Nervensystems. AIDS und AIDS-verwandte Zustände (ARC etc.), generalisierte Lymphadenopathie (PGL), Anti-HIV-Antikörper-Zustände, Kaposi-Sarkom und durch Viren ausgelöste Krebskrankheiten sind weitere Indikationen zur Anwendung von Verbindungen der Formel I.

Zum Gegenstand der vorliegenden Erfindung gehören weiterhin Arzneimittel mit einem Gehalt an mindestens einer Verbindung der Formel I, gegebenenfalls neben geeigneten Hilfs- und/oder Trägerstoffen.

Die Verbindungen der Formel I können entweder allein oder mit physiologisch verträglichen Hilfs- oder Trägerstoffen vermischt als Arzneimittel angewandt werden. Die Verbindungen der Formel I können oral in Dosen von vorzugsweise 1-50 mg/kg/Tag, besonders bevorzugt von 5-25 mg/kg/Tag appliziert werden. Die parenterale, rectale oder topische Anwendung oder als als Aerosol erfolgt vorzugsweise in einer Menge von 1-50 mg/kg/Tag, besonders bevorzugt von 5-25 mg/kg/Tag. Sie werden zweckmäßig in Dosierungseinheiten verabreicht, die mindestens die wirksame Menge der erfindungsgemäßen Verbindungen, bevorzugt 100-2500 mg, besonders bevorzugt 200-1250 mg enthalten. Diese Werte beziehen sich auf einen erwachsenen Menschen mit einem Gewicht von 75 kg. Die Dosierung kann in schweren Fällen auch erhöht werden. In vielen Fällen genügen jedoch auch geringere Dosen.

Die erfindungsgemäßen Verbindungen können auch in Kombination mit Immunstimulantien, wie Interferonen verabreicht werden.

Die Erfindung umfaßt weiterhin die Verwendung der erfindungsgemäßen Verbindungen bei der Herstellung von Arzneimitteln. Ein weiterer Gegenstand der Erfindung sind Arzneimittel, die eine oder mehrere Verbindungen der Formel I enthalten sowie Arzneimittel, die neben einer Verbindung der Formel I ein Immunstimulans enthalten.

Als Arzneimittel werden die pharmakologisch wirksamen Verbindungen der Formel I entweder als solche oder vorzugsweise in Kombination mit geeigneten pharmazeutischen Hilfs- und/oder Trägerstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 % beträgt.

Geeignete Hilfs- bzw. Trägerstoffe für die gewünschte Arzneimittelformulierung sind beispielsweise neben Lösemitteln, Gelbildnern, Suppositoriengrundlagen, Tabletten-Hilfsstoffen und anderen Wirkstoffträgern auch Antioxidantien, Dispergiermittel, Emulgatoren, Entschäumer, Geschmackskorrigentien, Konservierungsmittel, Lösungsvermittler oder Farbstoffe.

Die Wirkstoffe der Formel I können oral, parenteral, intravenös oder rectal appliziert werden, wobei neben der oralen Applikation insbesondere die intranasale Applikation als Aerosol bevorzugt ist. Für eine orale Anwendungsform werden die Verbindungen der Formel I mit den dafür geeigneten Zusatzstoffen wie Trägerstoffen, Stabilisatoren oder inerten Verdünnungsmitteln vermischt und durch die üblichen Methoden in geeignete Darreichungsformen gebracht, wie Tabletten, Dragees, Steckkapseln, wäßrige oder ölige Lösungen. Als inerte Trägerstoffe können z.B. Gummi arabicum, Magnesia, Magnesiumcarbonat, Kaliumphosphat, Milchzucker, Glucose oder Stärke, insbesondere Maisstärke verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran. Zur subcutanen oder intravenösen Applikation werden die Verbindungen der Formel I gegebenenfalls mit den dafür geeigneten Substanzen wie Lösungsvermittler, Emulgatoren oder weiteren Hilfsstoffen in Verbindung gebracht. Als Lösungsmittel kommen z.B. physiologische Kochsalzlösung oder Alkohole, z.B. Ethanol, Propanol, Glycerin, daneben auch Zuckerlösungen wie Glucose- oder Mannitlösungen, oder auch eine Mischung aus den verschiedenen Lösungsmitteln in Frage.

Die pharmazeutische Wirksamkeit der erfindungsgemäßen Verbindungen wird durch folgende Tests belegt.

I. Pathogenfreie NMRI-Mäuse (NMRI = Naval Medical Research Institute), weiblich, mit einem Gewicht von etwa 20 g werden mit Friend-Leukämie Virus-haltigem (FLV) Mäuseserum intravenös infiziert. 48 Stunden nach der Infektion beginnt eine 10 tägige Behandlung. Die Mäuse werden mit den in Tabelle 1 aufgeführten Verbindungen behandelt. Die intraperitoneale Applikation erfolgt in einer Dosis, während die orale Gabe über das Trinkwasser der Mäuse erfolgt. Am 14. Versuchstag werden die Tiere durch zervicale Dislocation getötet und die Milzen entnommen. Als Meßparameter der therapeutischen Wirksamkeit wird das Milzgewicht der behandelten Tiere mit dem der unbehandelten Kontrolltiere in Relation gesetzt. Als Standardsubstanz dient AZT.

**Tabelle 1**

| Verbindung | Applikationsart | Dosis mg/kg | Überlebensrate (%) | relatives Milzgewicht (%) |
|---|---|---|---|---|
| A | i.p. | 25 | 100 | 7.64 |
| AZT | i.p. | 50 | 100 | 5.81 |
| Kontrolle | i.p. | 0 | 100 | 11.22 |
| D | i.p. | 50 | 90 | 8.68 |
| AZT | i.p. | 50 | 100 | 7.54 |
| Kontrolle | i.p. | 0 | 100 | 11.36 |
| D | p.o. | 14 | 100 | 8.08 |
| AZT | p.o. | 18 | 100 | 6.42 |
| Kontrolle | p.o. | 0 | 100 | 11.36 |

II. Pathogenfreie NMRI-Mäuse mit einem Gewicht von etwa 16 g werden intranasal mit Influenza A2 infiziert und anschließend mit den in Tabelle 2 beschriebenen Verbindungen oral therapiert. Als Vergleich dient Amantadin. Die Behandlung erfolgt beginnend nach der Infektion zweimal täglich über 2,5 Tage. Der Behandlungserfolg wird anhand des Krankheitsverlaufes und der Überlebensrate gegenüber den unbehandelten Infektionskontrollen bestimmt. Diese Tiere erhalten anstelle der zu prüfenden Verbindungen eine wasserlösliche Methylhydroxyethylcellulose (Viskosität 300 Pa•S in 2 % Lösung) ((R)Tylose MH 300P) appliziert. Die Versuche werden mit Gruppen von je 5 Mäusen durchgeführt. Die chemotherapeutische Wirkung ist aus Tabelle 2 ersichtlich.

**Tabelle 2**

| Verbindung | Applikationsart | Dosis mg/kg | mittlere Überleb.zeit (Tage) | Überlebensrate (von 5) |
|---|---|---|---|---|
| D | p.o. | 0.25 | 7.7 | 2 |
| D | p.o. | 2.50 | 7.0 | 4 |
| Amantadin | p.o. | 80.00 | - | 5 |
| Kontrolle | p.o. | 0 | 8.0 | 1 |

**III**. Pathogenfreie NMRI-Mäuse mit einem Gewicht von etwa 15 g werden intraperitoneal mit Herpes simplex Typ 1 infiziert und anschließend mit den in Tabelle 3 genannten Verbindungen intraperitoneal therapiert. Die Behandlung erfolgt zweimal täglich über 2,5 Tage, beginnend nach der Infektion. Der Behandlungserfolg wird anhand des Krankheitsverlaufes und der Überlebensrate gegenüber den unbehandelten Infektionskontrollen bestimmt. Diese Tiere erhalten anstelle der zu prüfenden Verbindungen eine wasserlösliche Methylhydroxyethylcellulose (Viskosität 300 Pa•S in 2 % Lösung) ((R)Tylose MH 300P) appliziert. Die Versuche werden mit Gruppen von je 5 Mäusen pro Verbindung durchgeführt. Die chemotherapeutische Wirkung ist aus Tabelle 3 ersichtlich.

**Tabelle 3:**

| Verbindung | Applikationsart | Dosis mg/kg | mittlere Überleb.zeit (Tage) | Überlebensrate (von 5) |
|---|---|---|---|---|
| A | i.p. | 30 | 10 | 3 |
| Standard | i.p. | 30 | - | 5 |
| Kontrolle | i.p. | 0 | 8.6 | 0 |

**IV**. Die Prüfung von Präparaten gegen HIV in Zellkultur erfolgt mit Hilfe von Lymphozyten in RPMI-Medium pH 6.8, das zusätzlich noch 20 % foetales Kälberserum und 20 IU/ml rekombinantes Interleukin-2 enthält. Aus frischem Spenderblut mittels FicollR-Gradienten Zentrifugation isolierte Lymphozyten werden unter Zusatz von 2µg/ml Phytohämagglutinin (Wellcome) in komplettem Medium 36 Stunden bei 37° C unter 5 % $CO_2$ kultiviert. Die Zellen werden nach Zusatz von 10 % DMSO bei einer Zelldichte von $5x10^6$ eingefroren und in flüssigem Stickstoff gelagert. Für den Versuch werden die Zellen aufgetaut, in RPMI-Medium gewaschen und in komplettem Medium 3 bis 4 Tage kultiviert. Man löst die zu prüfenden Verbindungen in komplettem Medium. In 24-er Multiwell-Schalen werden jeweils 0.5 ml komplettes Medium gegeben. Nach Zugabe von 0.1 ml des gelösten Präparates wird durch Übertragung von jeweils 0.1 ml das Präparat in einer geometrischen Verdünnungsreihe, Faktor 5, verdünnt. Zu allen Ansätzen gibt man 0.4 ml einer Suspension mit $5x10^5$ Lymphozyten pro ml. Die Ansätze werden mit 0.1 ml eines Überstandes von mit HIV infizierten Lymphozyten versetzt, wobei die Multiplizität der infektiösen Einheit 0.01 bis 0.02 beträgt. Als Standard dient AZT. Die Auswertung erfolgt durch mikroskopische Betrachtung der Ansätze am vierten Tage nach der Infektion. Anhand der virusbedingten Syncytienbildung wird die Hemmung der Virusvermehrung bestimmt. Die Ergebnisse des Tests sind in Tabelle 4 zusammengefaßt.

**Tabelle 4:**

| Präparat | Erreger | Stamm | Wirkstärke | MHK (µg/ml) |
|---|---|---|---|---|
| A | HIV-1 | D34 | 30 | 75 |
| AZT | HIV-1 | D34 | 50 | 0.2 |
| Kontrolle | HIV-1 | D34 | 10 | >100 |
| A | HIV-1 | AR1 | 30 | 50 |
| Kontrolle | HIV-1 | AR1 | 10 | >100 |

Bewertungsskala: 10 unwirksam, 50 hochwirksam

AR1 ist ein AZT-resistenter Stamm, der in Gegenwart von AZT kultiviert wird.

Durch die nachfolgenden Synthesebeispiele soll die vorliegende Erfindung näher erläutert werden.

**Beispiel 1**: Synthese von 1-(5-Azido-2,3-didesoxy-β-D-xylofuranosyl-)thymin (A)

0.56 g (2,5 mmol) 3',5'-Anhydro-thymidin (Oxetan der Formel II) werden in 10 ml abs. DMF gelöst und diese Lösung mit 0.98 g (20 mmol) Lithiumazid versetzt. Nach 12-stündigem Rühren bei 120 °C ist dünnschichtchromatographisch kein Edukt mehr nachweisbar. Nach dem Abkühlen wird die Reaktionsmischung mit 10 ml Wasser versetzt, die Lösung am Rotationsverdampfer eingeengt und der resultierende Rückstand 2-mal mit Toluol koevaporiert. Die Reinigung des Rohproduktes erfolgt über eine Kieselgelsäule mit Methylenchlorid/Methanol (95:5). Nach dem Einengen und Trocknen erhält man 0.25 g eines amorphen Pulvers.

$^1$H-NMR ($d_6$-DMSO, 270 MHz) δ 1.8 (s, 3H, $CH_3$); 1.9 (m, 1H, H-2'); 2.6 (m, 1H, H-2''); 3.6 (m, 2H, H-5',H-5''); 3.95 (m,1H, H-4'); 5.55 (d, 1H, H-3'); 6.1 (g, 1H, h-1'); 7.8 s, 1H, H-6); 11.25 (s, 1H, N-H). UV (MeOH) ε = 9280 (265 nm). MS m/e 268 ($M+H^+$).

**Beispiel 2**: Synthese von 1-(3,5-Diazido-2,3,5-tridesoxy-β-D-xylofuranosyl-)thymin (D)

1,99 g (5 mmol) 3',5'-Di-O-Mesyl-thymidin werden in 25 ml abs. DMF gelöst und diese Lösung 140 Minuten bei 80 °C mit 1,96 g (40 mmol) Lithiumazid gerührt. Nach dem Abkühlen wird die Reaktionsmischung eingeengt und der Rückstand über eine Kieselgelsäule mit Cyclohexan/Essigester (1:1) gereinigt. Die dünnschichtchromatographisch einheitlichen Fraktionen ergeben nach dem Einengen 1,3 g farbloses Öl.

$^1$H-NMR ($CDCl_3$, 270 MHz) δ 1.9 (s, 3H, $CH_3$); 2.2 (m, 1H, H-2'); 2.8 (m, 1H, H-2''); 3.7 (m, 2H, H-5', H-5''); 4.1 (m, 1H, H-4'); 4.3 (m, 1H, H4'); 6.2 (q, 1H, H-1'); 7.4 (s, 1H, H-6); 8.85 (s, 1H, N-H). UV (MeOH) ε = 9260 (264 nm). MS m/e 293 ($M+H^+$).

**Patentansprüche**

1. Verbindung der Formel I

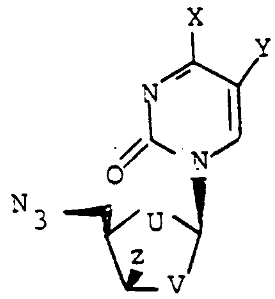

(I)

in der

X = H, OH, $OR^1$, $NH_2$, NH-CO-$R^2$, N-CO-$R^2$(CO-$R^3$)
Y = H, $C_1$-$C_4$-Alkyl, Halogen
Z = OH, $N_3$, F
U = O, S, $CH_2$ , $CF_2$, CHF
V = O, S, $CH_2$ oder eine direkte Bindung $C_1$-$C_3$

bedeuten, wobei $R^1$ - $R^3$ unabhängig voneinander geradkettige oder verzweigte Alkylgruppen mit 1 bis 20 Kohlenstoffatomen, Cycloalkylgruppen mit 3 bis 8 Kohlenstoffatomen, Aralkylgruppen mit 7 bis 20 Kohlenstoffatomen, Arylgruppen mit 6 bis 14 Kohlenstoffatomen sind, wobei $R^2$ und $R^3$ gegebenenfalls zusammen eine cyclische Imidstruktur bilden können.

**2.** Verbindungen der Formel I gemäß Anspruch 1, dadurch gekennzeichnet, daß

X = H, OH, $OR^1$, $NH_2$, NH-CO-$R^2$, N-CO-$R^2$ (CO-$R^3$)
Y = H, $C_1$-$C_4$-Alkyl, Halogen
Z = OH, $N_3$, F
U = O, S, $CH_2$, $CF_2$, CHF
V = $CH_2$

bedeuten, wobei $R^1$ - $R^3$ geradkettige oder verzweigte Alkylgruppen mit 1-6 C-Atomen, Cycloalkylgruppen mit 3-6 C-Atomen oder Aralkylgruppen mit 7-10 C-Atomen sind und wobei $R^2$ und $R^3$ gegebenenfalls zusammen eine cyclische Imidstruktur bilden können.

**3.** Verbindungen der Formel I gemäß den Ansprüchen 1 oder 2, dadurch gekennzeichnet, daß

X = H, OH, $NH_2$
Y = H, $CH_3$, $C_2H_5$, Cl
Z = OH, $N_3$
U = O, $CH_2$
V = $CH_2$

bedeuten,

**4.** Verfahren zur Herstellung einer Verbindung der Formel I gemäß einem oder mehreren der Ansprüche 1-3, dadurch gekennzeichnet, daß zur Herstellung der 5-Azido-3-hydroxy-$\beta$-D-xylofuranosyl-Derivate eine Verbindung der Formel II

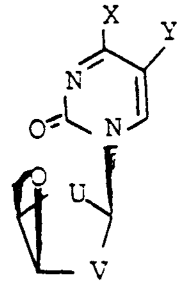

(II)

in der die Symbole die in den vorausgehenden Ansprüchen genannten Bedeutungen haben, mit Azid umgesetzt wird, oder zur Herstellung der 3,5-Diazido-β-D-xylofuranosyl-Derivate eine Verbindung der Formel III

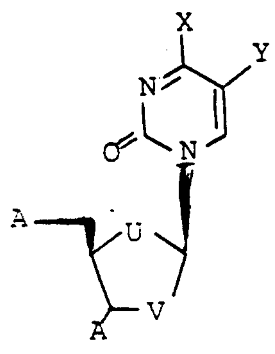

(III)

in der die Substituenten U-Y die in den vorausgehenden Ansprüchen genannten Bedeutungen haben und A eine Abgangsgruppe darstellt, mit Azid umgesetzt wird

oder zur Herstellung von 5-Azido-3-fluoro-β-D-xylofuranosyl-Derivaten eine Verbindung der Formel IV

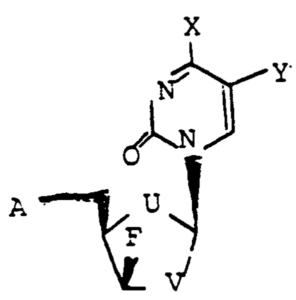

(IV)

in der die Symbole U-Y die in den vorausgehenden Ansprüchen genannten Bedeutungen haben und A eine Abgangsgruppe darstellt, mit Azid umgesetzt wird.

**5.** Verwendung einer Verbindung gemäß einem oder mehreren der Ansprüche 1-3 zur Herstellung von Arzneimitteln

**6.** Verwendung einer Verbindung gemäß einem oder mehreren der Ansprüche 1-3 zur Prophylaxe oder Behandlung von durch Viren verursachten Krankheiten.

**7.** Verfahren zur Prophylaxe oder Behandlung von durch Viren verursachten Krankheiten, dadurch gekennzeichnet, daß eine Verbindung gemäß einem oder mehreren der Ansprüche 1-3 verabreicht wird.

**8.** Arzneimittel, dadurch gekennzeichnet, daß sie mindestens eine Verbindung gemäß den Ansprüchen 1-3 enthalten.

**9.** Verfahren zur Herstellung von Arzneimitteln gemäß Anspruch 5, dadurch gekennzeichnet, daß mindestens eine Verbindung gemäß den Ansprüchen 1-3 gegebenenfalls mit geeigneten Hilfs- und/oder Trägerstoffen in eine geeignete Darreichungsform gebracht wird.

**10.** Arzneimittel, enthaltend neben einer Verbindung gemäß einem oder mehreren der Ansprüche 1-3 mindestens ein Immunstimulans.

**Patentansprüche für folgende Vertragsstaaten: ES GR**

**1.** Verfahren zur Herstellung einer Verbindung der Formel I

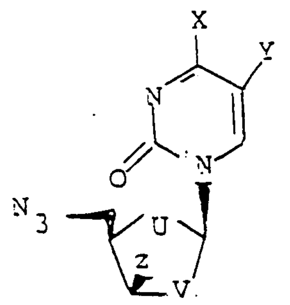

(I)

in der

X = H, OH, $OR^1$, $NH_2$, NH-CO-$R^2$, N-CO-$R^2$(CO-$R^3$)
Y = $C_1$-$C_4$-Alkyl, Halogen
Z = OH, $N_3$, F
U = O, S, $CH_2$
V = O, S, $CH_2$ oder eine direkte Bindung $C_1$-$C_3$

bedeuten, wobei $R^1$ - $R^3$ unabhängig voneinander geradkettige oder verzweigte Alkylgruppen mit 1 bis 20 Kohlenstoffatomen, Cycloalkylgruppen mit 3 bis 8 Kohlenstoffatomen Aralkylgruppen mit 7 bis 20 Kohlenstoffatomen Arylgruppen mit 6 bis 14 Kohlenstoffatomen sind, wobei $R^2$ und $R^3$ gegebenenfalls zusammen eine cyclische Imidstruktur bilden können, dadurch gekennzeichnet, daß zur Herstellung der 5-Azido-3-hydroxy-$\beta$-D-xylofuranosyl-Derivate eine Verbindung der Formel II

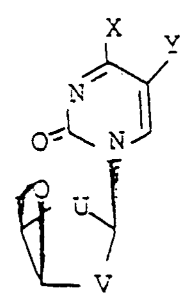

(II)

in der die Symbole die obengenannten Bedeutungen haben, mit Azid umgesetzt wird, oder zur Herstellung der 3,5-Diazido-$\beta$-D-xylofuranosyl-Derivate eine Verbindung der Formel III

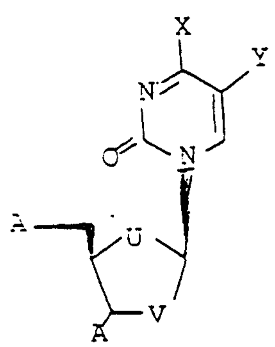

(III)

in der die Substituenten U-Y die obengenannten Bedeutungen haben und A eine Abgangsgruppe darstellt, mit Azid umgesetzt wird

oder zur Herstellung von 5-Azid-3-fluoro-β-D-xylofuranosyl-Derivate eine Verbindung der Formel IV

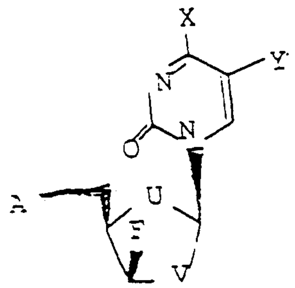

(IV)

in der die Symbole U-Y die obengenannten Bedeutungen haben und A eine Abgangsgruppe darstellt, mit Azid umgesetzt wird.

**2.** Verfahren zur Herstellung von Verbindungen der Formel I gemäß Anspruch 1, dadurch gekennzeichnet, daß
X = H, OH, $OR^1$, $NH_2$, $NH\text{-}CO\text{-}R^2$, $N\text{-}CO\text{-}R^2$ $(CO\text{-}R^3)$
Y = H, $C_1$-$C_4$-Alkyl, Halogen
Z = OH, $N_3$, F
U = O, S, $CH_2$, $CF_2$, CHF
V = $CH_2$
bedeuten, wobei $R^1$ - $R^3$ geradkettige oder verzweigte Alkylgruppen mit 1-6 C-Atomen, Cycloalkylgruppen mit 3-6 C-Atomen oder Aralkylgruppen mit 7-10 C-Atomen sind und wobei $R^2$ und $R^3$ gegebenenfalls zusammen eine cyclische Imidstruktur bilden können.

**3.** Verfahren zur Herstellung von Verbindungen der Formel I gemäß den Ansprüchen 1 oder 2, dadurch gekennzeichnet, daß
X = H, OH, $NH_2$
Y = H, $CH_3$, $C_2H_5$, Cl
Z = OH, $N_3$
U = O, $CH_2$
V = $CH_2$
bedeuten.

**4.** Verwendung einer Verbindung gemäß einem oder mehreren der Ansprüche 1-3 zur Herstellung von Arzneimitteln

**5.** Verwendung einer Verbindung gemäß einem oder mehreren der Ansprüche 1-3 zur Prophylaxe oder Behandlung von durch Viren verursachten Krankheiten.

**6.** Verfahren zur Prophylaxe oder Behandlung von durch Viren verursachten Krankheiten, dadurch gekennzeichnet, daß eine Verbindung gemäß einem oder mehreren der Ansprüche 1-3 verabreicht wird.

**7.** Arzneimittel, dadurch gekennzeichnet, daß sie mindestens eine Verbindung gemäß den Ansprüchen 1-3 enthalten.

**8.** Verfahren zur Herstellung von Arzneimitteln gemäß Anspruch 7, dadurch gekennzeichnet, daß mindestens eine Verbindung gemäß den Ansprüchen 1-3 gegebenenfalls mit geeigneten Hilfs- und/oder Trägerstoffen in eine geeignete Darreichungsform gebracht wird.

**9.** Arzneimittel, enthaltend neben einer Verbindung gemäß einem oder mehreren der Ansprüche 1-3 und mindestens ein Immunstimulans.

Europäisches Patentamt

**EUROPÄISCHER TEILRECHERCHENBERICHT,**
der nach Regel 45 des Europäischen Patentübereinkommens für das weitere Verfahren als europäischer Recherchenbericht gilt

Nummer der Anmeldung
EP 91110574.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X | DE - A1 - 3 643 091 (BAYER AG) * Zusammenfassung; Ansprüche * -- | 1,5,8 | C 07 H 19/06<br>A 61 K 31/70 |
| A | EP - A2 - 0 292 101 (THE WELLCOME FOUNDATION LTD.) * Zusammenfassung; Ansprüche * -- | 4,5,8 | |
| A | EP - A2 - 0 295 090 (THE WELLCOME FOUNDATION LTD.) * Zusammenfassung; Ansprüche * -- | 4 | |
| A | EP - A2 - 0 348 170 (LEE, MOSES NAM FONG) * Zusammenfassung; Ansprüche * ---- | 1,4 | |

RECHERCHIERTE SACHGEBIETE (Int. Cl.5)

C 07 H
A 61 K

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-5,8-10
Unvollständig recherchierte Patentansprüche: –
Nicht recherchierte Patentansprüche: 6,7
Grund für die Beschränkung der Recherche:

Artikel 52(4) EPÜ

| Recherchenort | Abschlußdatum der Recherche | Prüfer |
|---|---|---|
| WIEN | 19-09-1991 | SCHNASS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument